# EUROPEAN PATENT SPECIFICATION

(11) **EP 4 129 174 B1**
(45) Date of publication and mention of the grant of the patent: **04.11.2026**
(21) Application number: 20928471.0
(22) Date of filing: 16.07.2020
(51) Int. Cl.: A61B 5/153, A61B 5/00, A61B 8/08, A61B 6/03, A61B 5/055, A61B 5/15, A61M 5/42, A61B 34/20, A61B 34/30, A61B 17/34, A61M 5/32, A61M 5/46, A61B 8/00

(54) **AUTOMATIC BODY-INVASIVE DEVICE**
AUTOMATISCHE KÖRPERINVASIVE VORRICHTUNG
DISPOSITIF AUTOMATIQUE INVASIF POUR LE CORPS

(30) Priority: 02.04.2020 KR 20200040097
(43) Date of publication of application: 08.02.2023
(73) Proprietor: Airs Medical Co., Ltd., Seoul 08788 (KR)
(72) Inventor: LEE, Hye Seong, Seoul 06648 (KR); KIM, Yun Myeong, Seoul 06648 (KR)
(74) Representative: Isarpatent
(86) International application number: PCT/KR2020/009393
(87) International publication number: WO 2021/201343

(56) References cited:
- EP-A2- 2 170 204
- WO-A1-2008/081438
- WO-A1-2015/179505
- DE-A1- 102012 109 282
- JP-A- 2016 501 635
- JP-A- 2016 512 137
- KR-A- 20160 046 012
- KR-A- 20160 069 180
- KR-A- 20190 131 989
- US-A1- 2014 343 406
- US-A1- 2018 146 904
- BALTER MAX L. ET AL: "Adaptive Kinematic Control of a Robotic Venipuncture Device Based on Stereo Vision, Ultrasound, and Force Guidance", IEEE TRANSACTIONS ON INDUSTRIAL ELECTRONICS IEEE SERVICE CENTER, PISCATAWAY, NJ., USA, vol. 64, no. 2, 1 February 2017 (2017-02-01), pages 1626 - 1635, XP011638652, ISSN: 0278-0046, [retrieved on 20170110], DOI: 10.1109/TIE.2016.2557306

## Description

### TECHNICAL FIELD

The present invention relates to a body-insertion device. Specifically, the present invention relates to a device which moves an insertion unit to the location which is determined as a point of insertion through image analysis based on the image obtained by an image acquisition unit and then insert a medical instrument such as a venipuncture needle into the body at the location.

### BACKGROUND

**In** hospitals or clinics, a medical procedure that a medical instrument is inserted into a body are frequently performed for various purposes. For example, a venipuncture needle is inserted into a superficial vein for blood collection or intravenous injection and a catheter is inserted into some organs. In this specification, a venipuncture needle or blood collection needle is simply referred to as "needle" for brevity. Currently, a medical doctor, a nurse or a pathologist who is trained to find the location of blood vessel such as a superficial vein performs the insertion. The current system needs a long time for training the medical staffs and labor costs are incurred. Further, if a needle is not inserted at an appropriate location, the medical staff additionally tries to insert the needle into the body and a person whom the needle is inserted may experience unnecessary pain due to the additional needle insertion. Even a specialist has difficulties when inserting a needle into a person with poor blood vessel conditions such as a baby, an old person, a patient undergoing chemotherapy, a person having dark skin, and the like.

Repeated blood collection work can cause high fatigue and a lot of discomfort even for the experienced medical staffs. Further, a medical staff performing blood collection is exposed to infection risks when blood splashes onto the staff or enters the staff's body during the blood collection procedure.

The applicant filed a Korean Patent Application No. 10-2020-0010302 on January 29, 2020 for a method for exactly detecting a location of a target of a body such as a superficial vein by use of a machine learning. The application is an example of a method for detecting a location to be inserted by use of image analysis and machine learning.

### [Prior Art Reference]

Korean Patent No. 10-1601421 (Title: Automatic Method of Blood Collecting; Published on March 10, 2016)
Korean Patent No. 10-1362922 (Title: Module for Launching Blood Lancet and Apparatus having the Same)
International Patent Publication No. WO 2015/179505 A1
U.S. Patent Publication No. US 2014/343406 A1
EP Patent Publication No. EP 2 170 204 A2
International Patent Publication No. WO 2008/081438 A1
Japanese Patent Publication No. JP 2016 501635 A

Further prior art is disclosed in DE 10 2012 109282 A1 and BALTER MAX L. ET AL: "Adaptive Kinematic Control of a Robotic Venipuncture Device Based on Stereo Vision, Ultrasound, and Force Guidance",IEEE TRANSACTIONS ON INDUSTRIAL ELECTRONICS IEEE SERVICE CENTER, PISCATAWAY, NJ., USA, vol. 64, no. 2, 1 February 2017 (2017-02-01), pages 1626-1635, XP011638652,ISSN: 0278-0046, DOI: 10.1109/TIE.2016.2557306.

### DETAILED DESCRIPTION OF THE INVENTION

### TECHNICAL OBJECT

The object of the present invention is to provide a body-insertion device which detects an organ to be inserted and inserts a medical instrument into the organ.

### TECHNICAL SOLUTION

The present disclosure provides a body-insertion device for a body, which comprises a first moving unit for moving an image acquisition unit; an insertion unit; and a second moving unit for moving the insertion unit. The invention is defined by independent claim 1.

The first moving unit comprises at least one of a 1-1 module for moving the image acquisition unit in a 1-1 direction, a 1-2 module for moving the image acquisition unit in a 1-2 direction, and a 1-3 module for moving the image acquisition unit in a 1-3 direction.

The second moving unit comprises at least one of a 2-1 module for moving the insertion unit in a 2-1 direction, a 2-2 module for moving the insertion unit in a 2-2 direction, and a 2-3 module for moving the insertion unit in a 2-3 direction.

At least one of the 1-1 module to the 2-3 module can comprise a driving unit and an object to be driven by the driving unit. The object can be connected to the image acquisition unit or the insertion unit to move the unit in accordance with the control of the driving unit.

The object can be at least one of a timing belt extending between at least two pulleys driven by the driving unit, a screw driven by the driving unit, and rack/pinion driven by the driving unit.

The 1-1 direction can be a transvers direction of a body portion to be inserted; the 1-2 direction can be a height direction of the body portion; and the 1-3 direction can be a rotation direction which is a longitudinal direction of the body portion.

The 2-1 direction can be a transverse direction of a body portion to be inserted; the 2-2 direction can be a height direction of the body portion; and the 2-3 direction can be a direction having a predetermined angle with respect to a longitudinal direction of the body portion.

The device of the present invention can further comprise a 2-4 module for adjusting the predetermined angle, which rotates the insertion unit about at least one of the 2-1 direction and the 2-2 direction.

The image acquisition unit can be selected from a group consisting of an ultrasound probe, a photoacoustic imaging device, ToF sensor (time of flight sensor) comprising a near-infrared light emitting diode for recognizing the position coordinate of light reflected from the inside of a body, CT and MRI.

The body organ to be inserted can be blood vessel. The second moving unit can comprise a sensor for detecting inlet of blood.

The device of the present invention can further comprise at least one of a 1-4 module for rotating the image acquisition unit about the 1-2 direction, and a 1-5 module for moving the image acquisition unit along the longitudinal direction of a body portion to be inserted.

The device of the present invention can further comprise a position sensor for detecting the position of component which is moved by the first moving unit or the second moving unit and a distance sensor for measuring the relative distance between the components.

At least one of the components which is moved by the first moving unit or the second moving unit can comprise an electronic tag for recognition of position.

The present disclosure can also provide a non-claimed method for controlling the body-insertion device, which comprises a first step of moving the image acquisition unit to a body portion to be inserted by driving at least one of the 1-1 module to the 1-3 module; a second step of determining the location of the organ to be inserted based on the analysis of the image acquired by the image acquisition unit; and a third step of inserting a medical instrument of the insertion unit at the determined location by driving at least one of the 2-1 module to the 2-3 module.

The body organ to be inserted can be blood vessel or a nerve.

The method can further comprise a fourth step of recognizing an extending path of the organ by rotating the image acquisition unit about the 1-2 direction or moving the mage acquisition unit along the longitudinal direction of the body portion.

The method can further comprise a fifth step of measuring the pressure applied to the body portion by the image acquisition unit; and a sixth step of transmitting the measured pressure and the image corresponding to the measured pressure for using them in machine learning.

Methods described herein do not form part of the claimed invention.

### EFFECTS OF THE INVENTION

The present disclosure provides an effect of detecting the location to be inserted and moving the insertion body to the detected location for necessary medical procedure. In particular, the medical procedure according to the present invention can be carried out in easier and faster way than a medical staff's procedure. Further, the body-insertion device of the present disclosure can make a controlled pressed state for a blood vessel, thereby enabling the information on the controlled pressed state to be used in machine learning.

### BRIEF DECRIPTION OF DRAWINGS

FIG. 1 is a perspective view of the body-insertion device according to the present invention;
FIG. 2 is an exploded perspective view of the body-insertion device according to the present invention;
FIG. 3 is an exploded perspective view of a first moving unit of the present invention;
FIG. 4 is an exploded perspective view of a first moving unit of the present invention; and

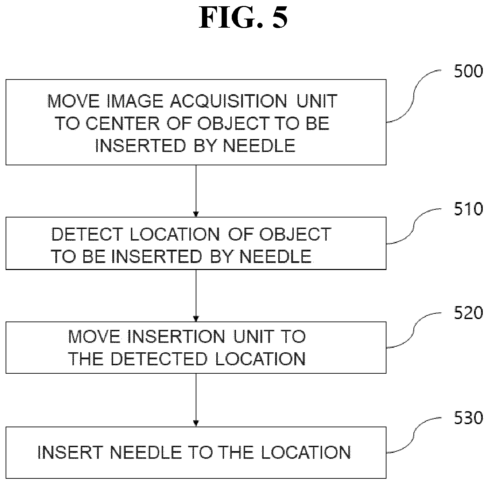

FIG. 5 is a flowchart of the body-insertion processes according to the present invention.

### EMBODIMENTS FOR CARRYING OUT THE INVENTION

Hereinafter, the present invention will be described in detail with reference to the accompanying drawings.

In this specification, transmitting / receiving of information (data) can be carried out with encryption /decryption if necessary. It should be understood that transmitting / receiving described in this specification can be carried out with encryption / decryption although not specifically mentioned. Further, transmitting (forwarding) to B from A or receiving by A from B include the process via an additional medium, not limited to direct transmitting or receiving. The order of each step should be understood in a non-limited manner unless a preceding step must be performed logically and temporally before a following step. That is, except for the exceptional cases as described above, although a process described as a following step is preceded by a process described as a preceding step, it does not affect the nature of the present invention, and the scope of rights should be defined regardless of the order of the steps. In addition, in this specification, "A or B" is defined not only as selectively referring to either A or B, but also as including both A and B. In addition, in this specification, the term "comprise" or "include" has a meaning of further including other components in addition to the components listed.

The term "module" or "unit" means a logical combination of a universal hardware and a software carrying out required function.

An aspect of the present invention can be an electronic arithmetic device such as a computer, server computer, smart phone, etc. An aspect of the present invention can be carried out by a computer program having codes which are conceived for carrying out the method. The computer program can be recorded in a computer-readable recording medium.

The term "value" described in this specification is defined as being universal value which includes vector, matrix, tensor and polynomial as well as scholar value.

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The components of the device can any shape, size, or dimension as well as those which are clearly illustrated in the appended drawings if they can perform the intended functions of the present invention. Further, the number of the components of the present invention can be differently selected as needed in the extent that it makes the device operable.

In this specification, the embodiments that the body-insertion device is used for a blood vessel are mainly described. However, it should be understood that the organ to be inserted by a medical instrument can be nerves, arteries, and the like and is not limited to the blood vessel.

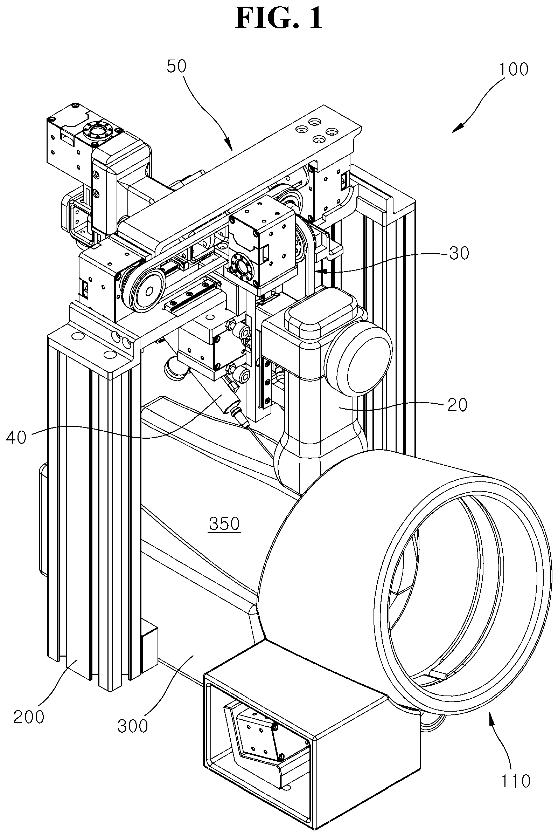

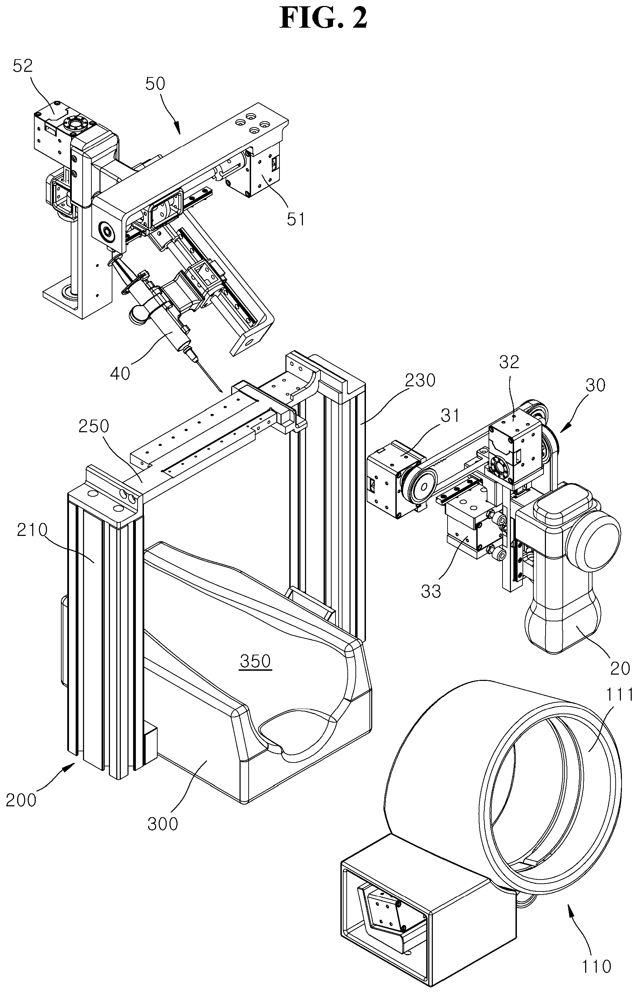

FIG. 1 is a perspective view of a body-insertion device (100) of the present invention; and FIG. 2 is an exploded perspective view of the device, where main units thereof are disassembled. The body-insertion device (100) comprises an image acquisition unit (20), a first driving unit (30) for moving the image acquisition unit (20), an insertion unit (40), a second driving unit (50) for moving the insertion unit (40), a body holding unit (110), a frame (200) and a body placing unit (300).

The image acquisition unit (20) is a unit for recognizing the inside of a body, for example, an ultrasound probe, a photoacoustic imaging device, a ToF (Time of Flight Sensor) which has a near-infrared light emitting diode for recognizing the position coordinate of light reflected from the inside of a body, CTs, MRIs, and the like. However, the image acquisition unit is not limited to the listed examples.

The body holding unit (110) holds the body portion while a needle is inserted. The body holding unit can hold an upper arm in the event that the device is used for collecting blood.

The frame (200) can comprise a pair of spaced posts (210, 230), a horizontal bar (250) which extends between the posts (210, 230). The horizontal bar (250) is provided with a first guide fixing portion (251) and a second guide fixing portion (253).

The body placing unit (300) is a unit where a body portion to be inserted by a needle is placed. A lower arm is placed thereto in the event that the device is used for collecting blood.

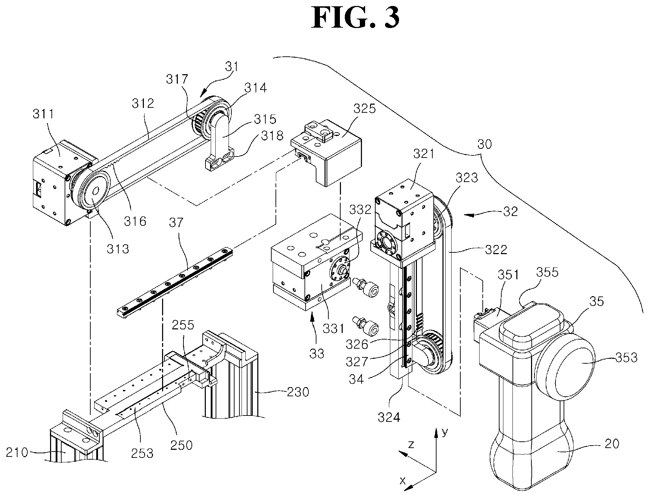

FIG. 3 is an exploded perspective view of the first moving unit (30). The first moving unit (30) comprises a 1-1 module (31) for moving the image acquisition unit (20) in a 1-1 direction, a 1-2 module (32) for moving the image acquisition unit (20) in a 1-2 direction, and a 1-3 module (33) for moving the image acquisition unit (20) in a 1-3 direction. Although it is illustrated and described that the modules are individually provided, they can be incorporated into one module. Alternatively, the modules can be constituted into two modules which includes a first module moving the image acquisition unit along two directions and a second module moving the image acquisition unit along the remaining one direction.

The 1-1 direction (x-axis direction) is the transverse direction of the body portion to be inserted by a needle; the 1-2 direction (y-axis direction) is the height direction of the body portion to be inserted by a needle; and the 1-3 direction is a rotation direction which is the longitudinal direction (z-axis direction) of the body portion to be inserted by a needle, i.e., the rotation direction on xy plane. The device (100) of the present invention can be used for collecting blood from blood vessels; can be used for intravenous injections; or can be used for inserting other medical parts such as a catheter into an organ. The device can be used for various medical purposes as well as the described ones

The 1-1 module (31) comprises a driving unit (311), a pair of spaced pulleys (313, 314), a timing belt (312) which extends between the pulleys, a fixing portion (315), a first connecting portion of timing belt (325), and a guide (37). The timing belt (312) is provided with a teeth portion (316). The pulleys (313, 314) are provided with a teeth portion (317) which can mesh with a teeth portion (316). The timing belt (322) and the pulleys (323, 327) of the 1-2 module (32) can be also provided with a teeth portion.

The term "driving unit" in this specification means a device for driving a driven object in a predetermined direction. It can be, for example, a motor, but is not limited to a specific device.

At least one of the pair of pulleys (313, 324) can be driven by the driving unit (311). The fixing portion (315) can be provided in at least one of the pair of pulleys (313, 314). For example, a mounting hole (318) provided under the fixing portion (315) and a mounting hole (255) provided on the horizontal bar (250) of the frame (200) are aligned with respect to each other and they are fastened by a bolt. The first connecting portion of timing belt (325) is connected to the timing belt (312) and is slidably provided on the guide (37). Further, the first connecting portion of timing belt (325) can be connected to the 1-3 module (33) as described in the below. The guide (37) extends along the 1-1 direction and can be fixed to a first guide fixing portion (253) of the horizontal bar (250).

In this specification, the term "a predetermined direction" should be construed to also include a direction having an angle with respect to the predetermined direction as well as the predetermined direction.

The driving unit (311) rotates the pulleys (313, 314), thereby causing the timing belt (312) to travel along the 1-1 direction (x direction), i.e., the transverse direction of the body portion to be inserted by a needle. The 1-3 module (33) which is connected to the timing belt (312) at the first connecting portion of timing belt (325) can move in the 1-1 direction along the guide (37). As described in the below, the image acquisition unit (20) can move in the 1-1 direction (x direction) by the 1-1 module (31) since the 1-3 module (33) is connected to the body (324) of the 1-2 module (32).

The 1-2 module (32) comprises a driving unit (321), a pair of spaced pulleys (323, 327), a timing belt which extends between the pair of pulleys (323, 327), a body (324), and a guide (34) provided in the body (324). At least one of the pair of pulleys (323, 327) can be driven by the driving unit (321). The guide (34) can extend on the body (324) along the 1-2 direction (y direction). The body (324) can be connected to the rotation axis (332) of the driving unit (331) of the 1-3 module (33) so as to rotate about z axis, i.e., rotate on the *xy* plane.

The device of the present invention can further comprise a holder (35) for holding the image acquisition unit (20). The holder (35) comprises a guide connecting portion (351) which is connected to the guide (34), a second connecting portion of timing belt (355) which is connected to the timing belt (322), and a fastening member (353) which fastens the image acquisition unit (20). The guide connecting portion (351) is provided to be slidable along with the guide (34).

The driving unit (321) rotates the pulleys (323, 327) so that the timing belt (322) travels along the 1-2 direction (y direction), i.e., the height direction of the body portion to be inserted by a needle. Then, the holder (35) which is connected to the timing belt (322) at the second connecting portion of timing belt (355) moves in the 1-2 direction along the guide (34). Consequently, the image acquisition unit (20) held by the holder (35) moves in the 1-2 direction.

The 1-3 module (33) comprises a driving unit (331) and an output shaft (332) thereof. The output shaft (332) is connected to the body (324) of the 1-2 module (32). When the driving unit (331) is turned on, the output shaft (332) rotates and then the body (324) can rotate about z axis, i.e., on the *xy* plane. When the body (324) rotates, the holder (35) connected to the body (324) and the image acquisition unit (20) can also rotate in the same direction.

In this specification, the expression 'rotate in a direction (or rotate about an axis)' and 'rotate on a plane' should be construed to also include 'rotate in a direction (or rotate about an axis) having an angle to the direction (or axis)' and 'rotate on a plane having an angle to the plane,' respectively.

The first moving unit (30) can further comprise an additional module (not illustrated) which moves the image acquisition unit (20) along z direction, i.e., the longitudinal direction of the body portion to be inserted by a needle and a rotating module (not illustrated) which rotates the image acquisition unit (20) on *xz* plane, i.e., about the 1-2 axis. The additional module can increase DOF (degree of freedom) of movement of the image acquisition unit (20) and help recognize the extending direction of blood vessel. The rotation module can also help recognize the extending direction of blood vessel as described in the below. The additional module can be incorporated into at least one of the 1-1 module (31) to the 1-3 module (33) or can be provided in a separate module.

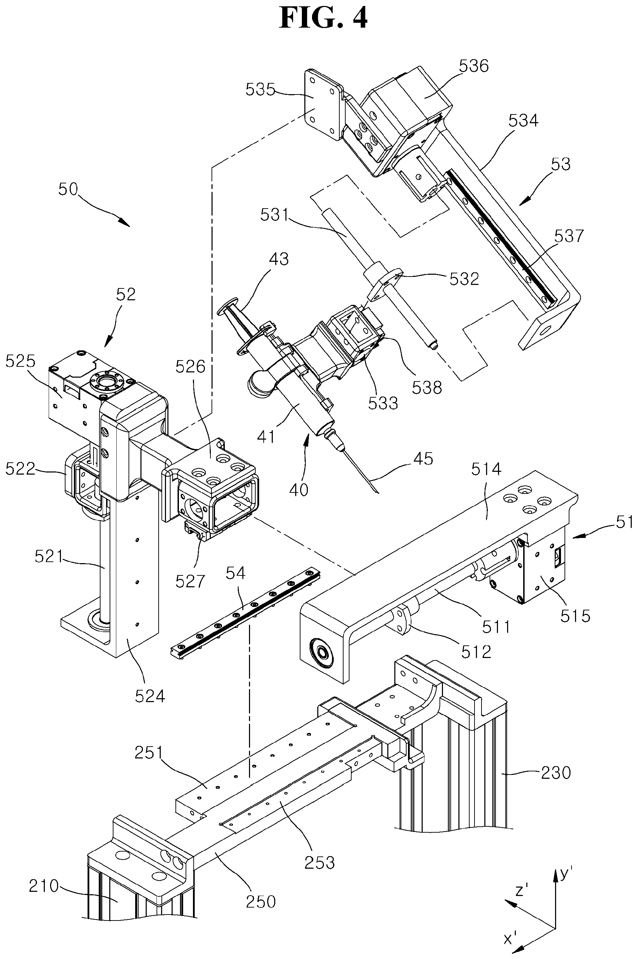

FIG. 4 shows an exploded perspective view of the second moving unit (50) of the device of the present invention.

The second moving unit (50) comprises a 2-1 module (51) which moves the insertion unit (40) in the 2-1 direction (*x*'), a 2-2 module (52) which moves the insertion unit (40) in the 2-2 direction (y'), and a 2-3 module (53) which moves the insertion unit (40) in the 2-3 direction (z'). The 2-3 direction can be the direction which has a predetermined angle to z' axis on the plane parallel to *y*'*z*' plane.

Although the modules (51, 52, 53) are individually drawn and described, they can be incorporated into one module. Alternatively, the modules can be constituted into two modules which includes a first module moving the insertion unit (40) in two directions and a second module moving the insertion unit in the remaining one direction.

The 2-1 direction (x'), the 2-2 direction (y'), z' direction can be defined the same as the 1-1 direction (x), the 1-2 direction (y) and the 1-3 direction (z), respectively. In the preferred embodiments, the 2-3 direction can have an angle with respect to the 1-3 direction. The angle can be 5° to 60°, preferably 20° to 45°.

The 2-1 module (51) comprises a screw (511), a movable member (512) which moves along the screw when the screw rotates, a body (514), a driving unit (515) and a guide (54). The screw (511) extends along the 2-1 direction (x' direction). The movable member (512) is fixed at the fixing portion (526). The fixing portion (526) comprises a guide mounting portion (527) thereunder, which is slidably connected to the guide (54). The fixing portion (526) can be provided at the body (524) of the 2-2 module (52). The guide (54) extends along the 2-1 direction and can be fixed at the second guide fixing portion (251) of the horizontal bar (250) of the frame (200).

The driving unit (515) rotates the screw (511), thereby moving the movable member (512) in the 2-1 direction on the screw (511). Then, the fixing portion (526) is guided along the guide (54) and moves in the 2-1 direction. Further, the body (524) can move in the 2-1 direction. As described in the below, the 2-3 module (53) where the insertion unit (40) is provided is fixed at the 2-2 module (52). Consequently, the driving unit (515) can move the insertion unit (40) in the 2-1 direction.

The 2-2 module (52) comprises a screw (521), a movable member (522) which moves on the screw when the screw rotates, a body (524), a driving unit (525) and a fixing portion (526). The movable member (522) is fixed at the fixing portion (535) of the 2-3 module (53), thereby connecting the 2-2 module (52) with the 2-3 module (53). The screw (521) extends along the 2-2 direction (y' direction). The body (524) can be provided with a guide (not illustrated) where the movable member (522) is slidably connected.

The driving unit (525) rotates the screw (521) so that the movable member (522) is guided on the screw (521) and moves in the 2-2 direction. Thus, the 2-3 module (53) connected to the movable member (522) also moves in the 2-2 direction; and then the insertion unit (40) provided at the 2-3 module (53) can also move in the 2-2 direction.

The 2-3 module (53) comprises a screw (531), a movable member (532), a body (534), a fixing portion (535), a driving unit (536), and a guide (537) which extends along the 2-3 direction and is provided at the body (534). The screw (531) extends along the 2-3 direction. The movable member (532) is fixed at the fixing portion (533) provided at the insertion unit (40). The insertion unit (40) comprises a body (41), a piston (43), a needle (45) which is inserted into the body, and a fixing portion (533). The fixing portion (533) is provided with a guide mounting portion (538) which is slidably connected to the guide (537). The insertion unit (40) can further comprise a sensor (not illustrated) which detects inlet of blood. The sensor can detect the inlet of blood by sensing blood color or blood absorbance. The sensor can be provided in the second moving unit (50), not in the insertion unit (40).

The driving unit (536) rotates the screw (531), thereby moving the movable member (532) in the 2-3 direction on the screw (531). Then, the insertion unit (40) connected to the movable member (532) moves in the 2-3 direction along the guide (537).

The second moving unit (52) can further comprise an adjustment module (not illustrated) for adjusting the angle of the insertion unit (40). The angle of the insertion unit (40) can be adjusted by rotating the insertion unit (40) about y' axis or by rotating the insertion unit (40) about x' axis, i.e., on *y'z'* plane. The adjustment by the former rotation can increase the accuracy of blood collecting since the insertion unit (40) can be aligned with the extending direction of blood vessel when the needle is inserted into the body. The adjustment by the latter rotation can adjust the penetration depth of needle. The rotation about y' axis and the rotation about x' axis can be performed by one module or two or more modules which are individually provided. Alternatively, at least one of the 2-1 module to the 2-3 module can be configured to perform the rotation.

The timing belt is used in the 1-1 module (31) and the 1-2 module (32) for easy measurement of pressure applied to the component to be driven. The screw is used in the 2-1 module (51) to 2-3 module (53) for positioning precision of the component to be driven. However, those embodiments are just exemplary, and the scope of the present invention is not limited to those embodiments. The screw can be used in at least one of the 1-1 module (31) to the 1-3 module (33) as the component to be driven. Further, the timing belt can be used in at least one of the 2-1 module (51) to the 2-3 module (53) as the component to be driven. Alternatively, rack and pinion (not illustrated) can be also used as the components to be driven and other various mechanical parts can be used as the component to be driven.

Further, an electronic tag (not illustrated) can be provided in at least one of the holder (35), the insertion unit (40), the guide connecting portion (351), the first connecting portion of timing belt (325) or the like. In those embodiments, the position of components can be easily calculated from the position of the electronic tag, which is detected by a sensor or a camera.

A controller (not illustrated) can calculate the position of the components of the device of the present invention and control the operation of the components. The controller can be an electronic arithmetic device connected to the device of the present invention, for example, PCs, smartphones, tablets, and the like or a remotely connected server.

The operation of the device of the present invention will be described. Although the embodiments wherein the purpose of insertion into the body is blood collecting and the image acquisition unit (20) is an ultrasound probe are described for convenience of explanation, it should be understood that the scope of the present invention is not limited to those embodiments. The operations described in the below can be carried out by the controller (not illustrated) in accordance with the program codes conceived for the present invention. The insertion position can be determined based on the conventional image analysis method and machine learning, in particular, Convolutional Neural Network technologies.

For blood collection, the location of a superficial vein is determined; the insertion unit (40) is moved to the determined location; a needle is inserted to the vein at the location; and then blood is drawn through the needle.

A person passes an arm through the holding unit (110) and then places the arm in the placing portion (350). Subsequently, in the step (500), at least one of the 1-1 module (31) and the 1-2 module (32) is driven to move the image acquisition unit (20) to the body portion to be inserted by a needle. It is preferred that at the first time the image acquisition unit (20) is moved to the center of the body portion.

In the step (510), the location of the organ to be inserted by a needle, for example, a superficial vein, is determined. The location can be determined by, for example, the methods disclosed in Korean Patent Application No. 10-2020-0010302 or other methods using the machine learning based on image analysis.

The image acquisition unit (20) is moved to the body portion to be inserted by a needle and comes into contact with the body portion. In an exemplary embodiment, the image acquisition unit (20) is moved in at least one of the 1-1 direction and the 1-3 direction to the location above the body portion. Subsequently, the driving unit (321) descend the image acquisition unit (20) in the 1-2 direction (y direction) so that the image acquisition unit comes into contact with the body portion. Whether the image acquisition unit (20) is in contact with the body portion can be recognized by sensing the load applied to the driving unit (321). In the event that the image acquisition unit (20) is an ultrasound probe, the pressure applied by the ultrasound probe can be controlled by adjusting the load applied to the driving unit (321). The pressure that the ultrasound probe applies to the body portion can be 0-50 mmHg, preferably 5-30 mmHg.

The ultrasonic probe can be maintained in the position where the size of the organ (for example, superficial vein) to be inserted by a needle is seen in the largest size. If the organ to be inserted by a needle is not recognized, the image acquisition unit (20) is moved to other location and the detection of the organ is resumed. At first, the detection is performed at the center of the body portion. If the organ to be inserted by a needle is not detected at the center, the image acquisition unit (20) can be moved to the lateral location from the center in the 1-1 direction and the detection at the location is performed. In the embodiments that the module for z direction is provided, if the second detection fails, the image acquisition unit (20) can be moved away from the center in z direction to perform further detection. If the detection fails a predetermined number of times, it can be treated as a failure of detecting superficial vein.

A portion of the ultrasound probe can be spaced apart from the body portion since the surface of the probe is plane unlike the body portion which is not plane. The 1-3 driving unit rotates the ultrasound probe (20) on *xy* plane so that the probe comes into contact with the body portion and then an image of the inside of the body can be obtained

The pressure applied to the body by the ultrasound probe can be measured and the measured pressure can be used in machine learning, thereby making it possible to perform machine learning about relationship among the optimal pressure, optimal pressing duration, optimal pressing location, and the like. Those data can be used as additional learning information for accurately classifying the anatomical structures observed in the ultrasound image. For example, the pressure where just a vein and subcutaneous fat are pressed, the pressure where muscle is pressed, the pressure where artery is pressed, and the like can be used as learning information, thereby increasing the accuracy of machine learning.

The accurate position of the image acquisition unit (20) and the insertion unit (40) should be obtained for operation of the device of the present invention.

The controller of the device can calculate the position of each component based on the dimension of the components and the control value of the driving unit (for example, output value). The method for calculating the position can be conceived from the conventional techniques and thus, the description thereof is omitted since it is not essential to the present invention.

The position of each component can be determined by a sensor or a camera. For example, the distance between the components can be determined by two or more cameras (not illustrated). Alternatively, the position can be determined by a distance sensor or a position sensor. The sensor can be an infrared sensor or a laser sensor. The distance sensor can measure the distance between the components which are moved by the driving unit. The position sensor detects whether the components are disposed at the predetermined position.

If the position calculated by the controller is different from that obtained by the sensor or the camera, the calculated position can be replaced by the position obtained by the sensor or the camera.

After the location of the superficial vein is determined by the image analysis and machine learning, the process of recognizing the extending direction of blood vessel can be performed. The extending direction of blood vessel can be recognized by analyzing the image obtained during the movement of the image acquisition unit (20) in the longitudinal direction of the body portion, i.e., z direction, or by analyzing the image obtained after the image acquisition unit (20) rotates about *y* axis. Additional module and/or rotation module (not illustrated) can be necessary for determining the extending direction of blood vessel.

It is not essential to recognize the extending direction of blood vessel in the present invention unlike the conventional blood collection performed by a medical staff. However, the recognition of the extending direction of blood vessel can increase the possibility of success of inserting needle.

In the step (520), after the superficial vein is identified by the above-mentioned processes, at least one of the 2-1 driving unit (515) to the 2-3 driving unit (536) moves the insertion unit (40) to the position close to the superficial vein. In the step (530), the needle (45) is further moved in the 2-3 direction; is inserted to the vein; and then the blood is collected by pulling a piston (43). A module for moving the piston (43) is not illustrated for brevity of illustration. A sensor for detecting the blood inflow to the insertion unit can be provided for detecting whether blood collection succeeds. It is preferred that the location of blood vessel is monitored during blood collection. Thus, it is preferred that the needle is inserted in the 2-3 direction while the ultrasound probe (20) is in contact with the object to be inserted by a needle.

According to the present invention, the inserting path of the needle (45) and the extending direction of blood vessel do not need to be on the same plane. **In** general, a medical staff who performs blood collection inserts a needle into a body at a small angle with respect to the skin with aligning the inserting path with the extending direction of blood vessel. It causes problems that the blood vessel is pushed, and the wall of blood vessel is scraped to be damaged. However, the needle can be inserted at a relatively large angle with respect to the skin and to the appropriate penetration depth since the image of blood vessel, the needle-inserting angle and the needle-location are monitored in real time through the analysis of the image obtained by the image acquisition unit (20). Further, the invasive range can be minimized according to the present invention.

Advantageously, the inserting angle of the needle (45) does not need to be aligned with the extending path of blood vessel and does not need to be disposed on the plane of the ultrasound probe.

**In** the event that the image acquisition unit (20) is a photoacoustic imaging device which does not require to be in contact with something, the needle (45) can be inserted into a blood vessel and collect blood in the state that the image acquisition unit (20) is not in contact with the body portion to be inserted by a needle. **In** that case, the needle (45) can be inserted at an angle which is nearly perpendicular to the skin, thereby more minimizing the invasive range.

## Claims

1. A body-insertion device (100) for a body comprising:
a body placing unit (300) on which a body portion being passed through a holding unit (110) is placed;
a frame (200) having a pair of pillar portions (210, 230) spaced in a transverse direction of the body portion being placed on the body placing unit (300), and a horizontal bar (250) extends between the pair of pillar portions (210, 230) in the transverse direction of the body portion;
an image acquisition unit (20);
a first moving unit (30) configured to move the image acquisition unit (20), and include a first guide (37) which extends in the transverse direction and fixed on a first guide fixing portion (251) of the horizontal bar (250);
an insertion unit (40); and
a second moving unit (50) is configured to move the insertion unit (40), and include a second guide (54)
which extends in the transverse direction and fixed on a second guide fixing portion (253) of the horizontal bar (250), wherein the second guide fixing portion (253) is configured to space from the first guide fixing portion (251) in direction of a longitudinal direction of the body portion being placed on the body placing unit (300);
wherein the first moving unit (30) comprises at least one of a 1-1 module (31) for moving the image acquisition unit in a 1-1 direction according to the first guide,
a 1-2 module (32) for moving the image acquisition unit in a 1-2 direction, and a 1-3 module (33) connected to the 1-1 module (31) for moving the image acquisition unit in a 1-3 direction; and
wherein the second moving unit (50) comprises at least one of a 2-1 module (51) for moving the insertion unit in a 2-1 direction according to the second guide,
a 2-2 module (52) for moving the insertion unit in a 2-2 direction, and a 2-3 module (53) for moving the insertion unit in a 2-3 direction, and
wherein the 1-1 direction and the 2-1 direction are a transversal direction of the body portion, and the 1-2 direction and the 2-2 direction are the height direction of a body portion, and the 1-3 direction is a direction of rotation from side to side around the longitudinal direction of the body portion as a reference axis, and the 2-3 direction is a direction having a predetermined angle with respect to the longitudinal direction of the body portion as the reference axis.

2. The device (100) according to claim 1, wherein at least one of the 1-1 module to the 2-3 module comprises a driving unit (30); and an object to be driven by the driving unit (30); wherein the object is connected to the image acquisition unit (20) or the insertion unit (40) to move the unit in accordance with the control of the driving unit (30); and wherein the object is at least one of a timing belt extending between at least two pulleys driven by the driving unit (30), a screw driven by the driving unit, and rack/pinion driven by the driving unit (30).

3. The device (100) according to claim 1, further comprising a 2-4 module for adjusting the predetermined angle, which rotates the insertion unit about at least one of the 2-1 direction and the 2-2 direction.

4. The device (100) according to any one of claims 1 to 3, wherein the image acquisition unit (20) is selected from a group consisting of an ultrasound probe, a photoacoustic imaging device, ToF sensor (time of flight sensor) comprising a near-infrared light emitting diode for recognizing the position coordinate of light reflected from the inside of a body, CT and MRI.

5. The device (100) according to any one of claims 1 to 3, wherein the body portion to be inserted is a blood vessel; and the second moving unit (50) comprises a sensor for detecting inlet of blood.

6. The device (100) according to any one of claims 1 to 3, further comprising at least one of a 1-4 module for rotating the image acquisition unit about the 1-2 direction, and a 1-5 module for moving the image acquisition unit (20) along the longitudinal direction of a body portion to be inserted.

7. The device (100) according to any one of claims 1 to 3, further comprising at least one of a position sensor for detecting the position of component which is moved by the first moving unit or the second moving unit and a distance sensor for measuring the relative distance between the components.

8. The device (100) according to any one of claims 1 to 3, wherein at least one of the components which is moved by the first moving unit or the second moving unit comprises an electronic tag for recognition of position.

## Patentansprüche

1. Körpereinführvorrichtung (100) für einen Körper, umfassend:
eine Körperplatzierungseinheit (300), auf der ein Körperabschnitt, der durch eine Halteeinheit (110) hindurchgeführt wird, platziert wird;
einen Rahmen (200), der ein Paar von Säulenabschnitten (210, 230) aufweist, die in einer Querrichtung des auf der Körperplatzierungseinheit (300) platzierten Körperabschnitts beabstandet sind, und eine horizontale Leiste (250) erstreckt sich zwischen dem Paar von Säulenabschnitten (210, 230) in der Querrichtung des Körperabschnitts;
eine Bildaufnahmeeinheit (20);
eine erste Bewegungseinheit (30), die dazu ausgebildet ist, die Bildaufnahmeeinheit (20) zu bewegen, und weist eine erste Führung (37) auf, die sich in der Querrichtung erstreckt und befestigt an einem ersten Führungsbefestigungsabschnitt (251) der horizontalen Leiste (250);
eine Einführeinheit (40); und
eine zweite Bewegungseinheit (50) ist dazu ausgebildet, die Einführeinheit (40) zu bewegen, und weist eine zweite Führung (54) auf, die sich in der Querrichtung erstreckt und befestigt an einem zweiten Führungsbefestigungsabschnitt (253) der horizontalen Leiste (250), wobei der zweite Führungsbefestigungsabschnitt (253) dazu ausgebildet ist, von dem ersten Führungsbefestigungsabschnitt (251) in Richtung einer Längsrichtung des auf der Körperplatzierungseinheit (300) platzierten Körperabschnitts beabstandet zu sein;
wobei die erste Bewegungseinheit (30) mindestens eines von einem 1-1-Modul (31) zum Bewegen der Bildaufnahmeeinheit in einer 1-1-Richtung gemäß der ersten Führung, einem 1-2-Modul (32) zum Bewegen der Bildaufnahmeeinheit in einer 1-2-Richtung und einem mit dem 1-1-Modul (31) verbundenen 1-3-Modul (33) zum Bewegen der Bildaufnahmeeinheit in einer 1-3-Richtung umfasst; und
wobei die zweite Bewegungseinheit (50) mindestens eines von einem 2-1-Modul (51) zum Bewegen der Einführeinheit in einer 2-1-Richtung gemäß der zweiten Führung, einem 2-2-Modul (52) zum Bewegen der Einführeinheit in einer 2-2-Richtung und einem 2-3-Modul (53) zum Bewegen der Einführeinheit in einer 2-3-Richtung umfasst, und
wobei die 1-1-Richtung und die 2-1-Richtung eine Querrichtung des Körperabschnitts sind, und die 1-2-Richtung und die 2-2-Richtung die Höhenrichtung eines Körperabschnitts sind, und die 1-3-Richtung eine Richtung einer Drehung von Seite zu Seite um die Längsrichtung des Körperabschnitts als eine Bezugsachse ist, und die 2-3-Richtung eine Richtung ist, die einen zuvor festgelegten Winkel in Bezug auf die Längsrichtung des Körperabschnitts als die Bezugsachse aufweist.

2. Vorrichtung (100) nach Anspruch 1, wobei mindestens eines von dem 1-1-Modul bis zu dem 2-3-Modul eine Antriebseinheit (30); und ein durch die Antriebseinheit (30) anzutreibendes Objekt umfasst; wobei das Objekt mit der Bildaufnahmeeinheit (20) oder der Einführeinheit (40) verbunden ist, um die Einheit gemäß der Steuerung der Antriebseinheit (30) zu bewegen; und wobei das Objekt mindestens eines ist von einem Steuerungszahnriemen, der sich zwischen mindestens zwei durch die Antriebseinheit (30) angetriebenen Riemenscheiben erstreckt, einer Schnecke, die durch die Antriebseinheit angetrieben wird, und einem Zahnstangengetriebe, das durch die Antriebseinheit (30) angetrieben wird.

3. Vorrichtung (100) nach Anspruch 1, des Weiteren umfassend ein 2-4-Modul zum Einstellen des zuvor festgelegten Winkels, das die Einführeinheit um mindestens eine der 2-1-Richtung und der 2-2-Richtung dreht.

4. Vorrichtung (100) nach einem der Ansprüche 1 bis 3, wobei die Bildaufnahmeeinheit (20) ausgewählt ist aus einer Gruppe bestehend aus einer Ultraschallsonde, einer photoakustischen Bildgebungsvorrichtung, einem ToF-Sensor (Time-of-Flight-Sensor), der eine Nahe-Infrarot-Leuchtdiode zum Erkennen der Positionskoordinate von aus dem Inneren eines Körpers reflektiertem Licht umfasst, CT und MRT.

5. Vorrichtung (100) nach einem der Ansprüche 1 bis 3, wobei der einzuführende Körperabschnitt ein Blutgefäß ist; und die zweite Bewegungseinheit (50) einen Sensor zum Detektieren des Einströmens von Blut umfasst.

6. Vorrichtung (100) nach einem der Ansprüche 1 bis 3, des Weiteren umfassend mindestens eines von einem 1-4-Modul zum Drehen der Bildaufnahmeeinheit um die 1-2-Richtung und einem 1-5-Modul zum Bewegen der Bildaufnahmeeinheit (20) entlang der Längsrichtung eines einzuführenden Körperabschnitts.

7. Vorrichtung (100) nach einem der Ansprüche 1 bis 3, des Weiteren umfassend mindestens eines von einem Positionssensor zum Detektieren der Position einer Komponente, die durch die erste Bewegungseinheit oder die zweite Bewegungseinheit bewegt wird, und einem Distanzsensor zum Messen der relativen Distanz zwischen den Komponenten.

8. Vorrichtung (100) nach einem der Ansprüche 1 bis 3, wobei mindestens eine der Komponenten, die durch die erste Bewegungseinheit oder die zweite Bewegungseinheit bewegt wird, einen elektronischen Transponder zum Erkennen der Position umfasst.

## Revendications

1. Dispositif d'insertion corporelle (100) pour un corps comprenant :
une unité de placement corporel (300) sur laquelle est placée une partie corporelle étant passée à travers une unité de support (110) ;
un bâti (200) possédant une paire de parties de montant (210, 230) espacées dans une direction transversale de la partie corporelle étant placée sur l'unité de placement corporel (300), et une barre horizontale (250) s'étend entre la paire de parties de montant (210, 230) dans la direction transversale de la partie corporelle ;
une unité d'acquisition d'images (20) ;
une première unité de déplacement (30) configurée pour déplacer l'unité d'acquisition d'images (20), et comporte un premier guide (37) qui s'étend dans la direction transversale et fixé sur une première partie de fixation de guide (251) de la barre horizontale (250) ;
une unité d'insertion (40) ; et
une seconde unité de déplacement (50) est configurée pour déplacer l'unité d'insertion (40), et comporte un second guide (54) qui s'étend dans la direction transversale et fixé sur une seconde partie de fixation de guide (253) de la barre horizontale (250), dans lequel la seconde partie de fixation de guide (253) est configurée pour être espacée de la première partie de fixation de guide (251) en direction d'une direction longitudinale de la partie corporelle étant placée sur l'unité de placement corporel (300) ;
dans lequel la première unité de déplacement (30) comprend au moins un parmi un module 1-1 (31) pour déplacer l'unité d'acquisition d'images dans une direction 1-1 en fonction du premier guide, un module 1-2 (32) pour déplacer l'unité d'acquisition d'images dans une direction 1-2, et un module 1-3 (33) relié au module 1-1 (31) pour déplacer l'unité d'acquisition d'images dans une direction 1-3 ; et
dans lequel la seconde unité de déplacement (50) comprend au moins un parmi un module 2-1 (51) pour déplacer l'unité d'insertion dans une direction 2-1 en fonction du second guide, un module 2-2 (52) pour déplacer l'unité d'insertion dans une direction 2-2, et un module 2-3 (53) pour déplacer l'unité d'insertion dans une direction 2-3, et
dans lequel la direction 1-1 et la direction 2-1 sont une direction transversale de la partie corporelle, et la direction 1-2 et la direction 2-2 sont la direction de hauteur d'une partie corporelle, et la direction 1-3 est une direction de rotation d'un côté à l'autre autour de la direction longitudinale de la partie corporelle en tant qu'axe de référence, et la direction 2-3 est une direction ayant un angle prédéterminé par rapport à la direction longitudinale de la partie corporelle en tant qu'axe de référence.

2. Dispositif (100) selon la revendication 1, dans lequel au moins un du module 1-1 au module 2-3 comprend une unité d'entraînement (30) ; et un objet à entraîner par l'unité d'entraînement (30) ; dans lequel l'objet est relié à l'unité d'acquisition d'images (20) ou à l'unité d'insertion (40) pour déplacer l'unité en fonction de la commande de l'unité d'entraînement (30) ; et dans lequel l'objet est au moins une parmi une courroie crantée s'étendant entre au moins deux poulies entraînées par l'unité d'entraînement (30), une vis entraînée par l'unité d'entraînement, et une crémaillère/un pignon entraîné par l'unité d'entraînement (30).

3. Dispositif (100) selon la revendication 1, comprenant en outre un module 2-4 pour régler l'angle prédéterminé, qui fait tourner l'unité d'insertion autour d'au moins une parmi la direction 2-1 et la direction 2-2.

4. Dispositif (100) selon l'une quelconque des revendications 1 à 3, dans lequel l'unité d'acquisition d'images (20) est choisie parmi un groupe constitué d'une sonde ultrasonore, d'un dispositif d'imagerie photoacoustique, d'un capteur ToF (capteur de temps de vol) comprenant une diode électroluminescente dans l'infrarouge proche pour reconnaître la coordonnée de position de la lumière réfléchie depuis l'intérieur d'un corps, CT et IRM.

5. Dispositif (100) selon l'une quelconque des revendications 1 à 3, dans lequel la partie corporelle à insérer est un vaisseau sanguin ; et la seconde unité de déplacement (50) comprend un capteur pour détecter une entrée de sang.

6. Dispositif (100) selon l'une quelconque des revendications 1 à 3, comprenant en outre au moins un parmi un module 1-4 pour faire tourner l'unité d'acquisition d'images autour de la direction 1-2 et un module 1-5 pour déplacer l'unité d'acquisition d'images (20) le long de la direction longitudinale d'une partie corporelle à insérer.

7. Dispositif (100) selon l'une quelconque des revendications 1 à 3, comprenant en outre au moins un parmi un capteur de position pour détecter la position d'un composant qui est déplacé par la première unité de déplacement ou la seconde unité de déplacement, et un capteur de distance pour mesurer la distance relative entre les composants.

8. Dispositif (100) selon l'une quelconque des revendications 1 à 3, dans lequel au moins l'un des composants qui est déplacé par la première unité de déplacement ou la seconde unité de déplacement comprend une étiquette électronique pour une reconnaissance de position.
